# EUROPÄISCHE PATENTSCHRIFT

(11) **EP 3 766 961 B1**
(45) Veröffentlichungstag und Bekanntmachung des Hinweises auf die Patenterteilung: **28.12.2022**
(21) Anmeldenummer: 19187072.4
(22) Anmeldetag: 18.07.2019
(51) Int. Cl.: C12N 5/00, B33Y 80/00

(54) **VERFAHREN ZUR ERZEUGUNG VON BIOLOGISCHEM GEWEBE**
METHOD FOR PRODUCING BIOLOGICAL TISSUE
PROCÉDÉ DE PRODUCTION DE TISSU BIOLOGIQUE

(43) Veröffentlichungstag der Anmeldung: 20.01.2021
(73) Patentinhaber: Axenoll Life Sciences AG, 8001 Zurich (CH)
(72) Erfinder: DECK, David L., 8001 Zürich (CH); VASIC, Srdan, 8001 Zürich (CH)
(74) Vertreter: Wenzel Nemetzade Warthmüller Patentanwälte Part mbB

(56) Entgegenhaltungen:
- EP-A1- 3 482 934
- WO-A1-2019/129361
- US-A1- 2019 187 128
- Anonymous: "Biologe*in/Biotechnologe*in /Biochemiker*in (m/w/d) {Biologe/Biologin} - Axenoll 3D Printing GmbH", , 20. Juni 2019 (2019-06-20), XP055648996, Gefunden im Internet: URL:http://dokumente.anzeigendaten.de/doku server/anzeigen/2019/06/20/07/O-SIS_10000- 1170656166-S.htm [gefunden am 2019-12-04]
- AHU ARSLAN-YILDIZ ET AL: "Towards artificial tissue models: past, present, and future of 3D bioprinting", BIOFABRICATION, Bd. 8, Nr. 1, 1. März 2016 (2016-03-01), Seite 014103, XP055648999, DOI: 10.1088/1758-5090/8/1/014103
- SOONHEE MOON ET AL: "Spatial Control of Bacteria Using Screen Printing", 3D PRINTING AND ADDITIVE MANUFACTURING, Bd. 3, Nr. 4, 1. Dezember 2016 (2016-12-01), Seiten 194-203, XP055648876, ISSN: 2329-7662, DOI: 10.1089/3dp.2016.0040

## Beschreibung

Die Erfindung betrifft ein Verfahren zur Erzeugung von biologischem Gewebe. Ebenso betrifft die Erfindung biologisches Gewebe, das nach einem solchen Verfahren erzeugt ist.

Es ist bekannt Gewebe beispielsweise im Wege des Tintenstrahldruckens herzustellen. Hierzu können Druckmedien mit lebenden Zellen zum Einsatz kommen. Dadurch können die Zellen jedoch regelmäßig hoher mechanischer Belastungen ausgesetzt sein. Je nach Ausgestaltung der Drucksysteme können die Zellen insbesondere hohen Scher- und/oder Druckbelastungen und/oder Reibungsbelastungen ausgesetzt werden. Die Überlebensrate der Zellen kann sich hierdurch verringern und das jeweils erzeugte Zellgewebe damit beeinträchtigt werden.

Ferner geht das Tintenstrahldrucken von Zellen beziehungsweise von Druckmedien mit darin enthaltenen Zellen mit dem Nachteil einher, dass 2-dimensionale Anordnungen nur sequentiell aufgebaut werden können. Ein dreidimensionaler Druck im Wege des Tintenstrahldruckens ist entsprechend mit noch längeren Herstellzeiten verbunden. Derartig lange Druck- beziehungsweise Herstellzeiten beeinträchtigen die Wirtschaftlichkeit. Gleichzeitig kann durch lange Druck- beziehungsweise Herstellzeiten die Überlebensrate der Zellen negativ beeinflusst werden, da sich lebende Zellen nur beschränkt Teilen können. Bei mehreren bereits erfolgten Zellteilungen bis zur Fertigstellung eines gesamten Drucks kann die weitere Überlebensfähigkeit der Zellen zeitlich stark begrenzt sein.

Der Stand der Technik in der Druckschrift US 2019/187128 A1 betrifft ein Verfahren zur Herstellung eines multilamellaren Gewebemodells. Das Verfahren umfasst das Positionieren eines ersten Siebes mit einem ersten Muster über einem Substrat, das Aufbringen einer ersten zu druckenden Lösung auf das erste Sieb, wobei die erste Lösung ein Hydrogel und gegebenenfalls einen zweiten Bestandteil umfasst, das Verschieben eines Rakels über das erste Sieb, um die erste Lösung in das erste Muster einzubringen, das Entfernen des ersten Siebs, um eine erste Schicht freizulegen, das Positionieren eines zweiten Siebs mit einem zweiten Muster über der ersten Schicht, das Platzieren einer zweiten zu druckenden Lösung auf dem zweiten Sieb, das Schieben eines Rakels über das zweite Sieb, um die zweite Lösung in das belichtete zweite Muster einzubringen und das Entfernen des zweiten Siebs, um eine zweite Schicht freizulegen, die auf der ersten Schicht positioniert ist. Gemäß US 2019/187128 A1 hergestellte Modelle sind für Hochdurchsatz-Screening geeignet. Sie ermöglichen die Untersuchung des Krankheitsverlaufs und können für Screening-Therapien verwendet werden.

In einer Stellenausschreibung "Biologe*in/Biotechnologe*in /Biochemiker*in (m/w/d)" der Axenoll 3D Printing GmbH vom 20. Juni 2019 wird das sogenannte 3D Siebdruckverfahren erwähnt, welches individualisierte Geometrien in Großserie bei freier Materialauswahl ermöglichen soll. Diese freie Materialwahl im Bioprinting soll Elemente wie Matrizes, Scaffolds, Zellen und Kombinationen davon, wie zum Beispiel Gewebe, Organoide, Organe, umfassen.

Der Fachartikel "Towards artificial tissue models: past, present, and future of 3D bioprinting" von Ahu Arslan-Yildiz et. al., in Biofabriaction, Vol. 8, Nr. 1, betrifft die regenerative Medizin und das sogenannte Tissue-Engineering. Gemäß dieser Publikation sollen mit der Bioprinting-Technologie künstliche Gewebe und Organe hergestellt werden, indem Gerüste mit kontrollierter räumlicher Heterogenität der physikalischen Eigenschaften, der zellulären Zusammensetzung und der Organisation der extrazellulären Matrix produziert werden.

Die Publikation "Spatial Control of Bacteria Using Screen Printing" von Soonhee Moon, et. al. in 3D Printing and Additive Manufacturing, Vol. 3, Nr. 4, betrifft den Einsatz einer Siebdruckmethode, um Bakterien auf Agar-, Glas- und Papieroberflächen zu strukturieren.

Die Druckschrift EP 3 482 934 A1 betrifft ein Drucksystem zum Drucken dreidimensional geformter Strukturen, insbesondere auf Substraten wie Leiterplatten, Wafer, Solarzellen, Trägersubstraten, Drucktischen, Trägerplatten, Sinterplatten oder dergleichen, mit zumindest einer Druckvorrichtung, die einen Druckkopf aufweist, mit einer Rakeleinrichtung und einer der Rakeleinrichtung zugeordneten Siebaufnahme zur Aufnahme eines Drucksiebs.

Die Druckschrift WO 2019/129361 A1 betrifft ein Verfahren zur Herstellung eines Arzneimittelabgabesystems. Das Verfahren umfasst die Schritte des Siebdruckens einer Basispaste und des Aushärtens der Basispaste. Weiterhin umfasst das Verfahren die Schritte des Siebdruckens einer ersten Paste getrennt von der Basispaste und des Härtens der ersten Paste.

Vor dem oben dargelegten Hintergrund bestand die Aufgabe der vorliegenden Erfindung darin, ein Verfahren zur Erzeugung von biologischem Gewebe anzugeben, das mit verbesserter Produktivität und gleichzeitig verringerter Gefahr von Zellbeschädigungen durchgeführt werden kann.

Diese Aufgabe ist mit dem Gegenstand des Anspruchs 1 gelöst worden. Vorteilhafte Ausgestaltungen sind Gegenstand der abhängigen Ansprüche und werden nachfolgend erläutert.

Bei einem erfindungsgemäßen Verfahren zur Erzeugung von biologischem Gewebe wird eine Unterlage für Zellgewebe bereitgestellt, ein lebende Zellen enthaltenes Druckmedium bereitgestellt, das Druckmedium durch ein Drucksieb und/oder eine Druckschablone auf die Unterlage gedruckt und die gedruckten Zellen entwickeln sich durch den Druck und/oder nach dem Druck zu Gewebe.

Durch ein erfindungsgemäßes Verfahren erzeugtes Gewebe eignet sich insbesondere für die Durchführung medizinischer und/oder pharmakologischer Untersuchungen und/oder Behandlungen. Insbesondere kann durch ein erfindungsgemäßes Verfahren erzeugtes Gewebe für in vitro Untersuchungen, bevorzugt für analytische Zwecke, und/oder für in vivo Behandlungen, bevorzugt für medizinische Zwecke, eingesetzt werden.

Durch das Drucken des Druckmediums durch ein Drucksieb und/oder eine Druckschablone kann in vorteilhafter Weise ein hohes Maß an Produktivität gewährleistet werden. Insbesondere erlaubt die Ausgestaltung des Drucksiebs und/oder der Druckschablone das Drucken definierter Druckbereiche beziehungsweise gesamter Drucklagen und/oder Gewebelagen in nur einem Druckschritt. Der gesamte Druckprozess kann somit in einem nur geringen Zeitraum abgeschlossen werden. Der verbleibende Überlebenszeitraum der jeweiligen Zellen kann dadurch verlängert werden.

Gleichzeitig kann das Drucksieb beziehungsweise die Druckschablone in vorteilhafter Weise die Scherbelastungen, Druckbelastungen und/oder Reibungsbelastungen der Zellen im Druckmedium reduzieren. Öffnungen des Drucksiebs und/oder der Druckschablone können im Hinblick auf verringerte mechanische Belastungen der Zellen, wie Scherbelastungen, Druckbelastungen und/oder Reibungsbelastungen, in ausreichender Größe ausgestaltet sein. Die Gefahr von Beschädigungen der Zellen im Druckmedium durch den Vorgang des Druckens beziehungsweise Auftragens auf die Unterlage kann hierdurch verringert werden. Die Eignung des erfindungsgemäß erzeugten Gewebes für medizinische Behandlungen und/oder pharmakologische Untersuchungen wird dadurch verbessert. Insbesondere ermöglicht das Drucken des lebende Zellen enthaltenden Druckmediums durch ein Drucksieb und/oder durch eine Druckschablone auf eine Unterlage die besonders vorteilhafte Entwicklung des Druckmediums zu Gewebe.

Unter Gewebe soll vorliegend Zellgewebe verstanden werden, insbesondere aus Zellen bestehendes und/oder Zellen enthaltendes biologisches Gewebe.

Unter Unterlage kann hier eine maschinenseitige Unterlage beziehungsweise an einer Druckanlage vorgesehene, insbesondere fest vorgesehene, Unterlage verstanden werden. Ebenso kann es sich bei einer Unterlage um eine Trägerstruktur handeln, die nur für einen Druckvorgang oder bis zum Abschluss des Druckens in einer Druckanlage positioniert wird und dann gemeinsam mit dem gedruckten Objekt aus der jeweiligen Druckanlage entnommen wird. Es kann sich bei der Trägerstruktur beispielsweise um einen Objektträger und/oder eine Zellkulturplatte handeln. Eine solche Trägerstruktur eignet sich in besonders vorteilhafter Weise für die Verwendung und/oder Handhabung in Laboratorien beziehungsweise für standardisierte Transportbehältnisse, Transportvorrichtungen und/oder Aufbewahrungsvorrichtungen.

Im Sinne der vorliegenden Erfindung kann ein Drucksieb mit einem Gewebe ausgestattet oder durch ein Gewebe und/oder durch einen Rahmen für das Gewebe gebildet sein. Ein solches Gewebe kann für das Hindurchdrücken einer Druckpaste ausgebildet sein. Das Gewebe eines Drucksiebs kann in einem Rahmen angeordnet oder eingespannt sein. Ferner kann ein Gewebe durch Metall- und/oder Kunststofffäden gebildet sein. Solche Metall- und/oder Kunststofffäden können verdrillt angeordnet sein oder einzeln verlaufen.

Zur Erzielung der jeweils gewünschten Druckform kann ein Drucksieb durchlässige und undurchlässige Bereiche aufweisen. Durchlässige Bereiche können für ein Druckmedium durchlässig und undurchlässige Bereiche können für ein Druckmedium undurchlässig sein. Durchlässige Bereiche eines Drucksiebs können im Sinne der vorliegenden Erfindung einen Durchlass und/oder einen Ausschnitt bilden, in dem ein für ein Druckmedium durchlässiges Gewebe angeordnet ist. Undurchlässige Bereiche können ebenfalls mit einem Gewebe versehen sein, welches jedoch für ein Druckmedium undurchlässig ausgestaltet oder zur Erzielung einer Undurchlässigkeit bedeckt ist.

Mit einem Drucksieb können feine Strukturen erzeugt werden, insbesondere mit einer Dicke zwischen 15 µm und 500 µm. Der Druck mittels eines Drucksiebs kann mit verhältnismäßig hoher Genauigkeit erfolgen, insbesondere mit einer hohen Genauigkeit in den Randbereichen des Druckerzeugnisses.

Im Sinne der vorliegenden Erfindung kann eine Druckschablone mit Öffnungen und/oder Ausschnitten ausgestattet sein, insbesondere mit Durchgangsöffnungen und/oder Ausschnitten, die in eine Platte eingebracht sind. Eine Platte mit Öffnungen und/oder Ausschnitten kann eine Druckschablone im Sinne der vorliegenden Erfindung bilden. Öffnungen und/oder Ausschnitte können dementsprechend einen Durchlass für Druckpaste bilden. Durch solche Öffnungen und/oder Ausschnitte kann im Rahmen eines Druckprozesses Druckpaste hindurchgedrückt werden. Eine Druckschablone kann insbesondere frei von Gewebe ausgebildet sein. Insbesondere können die Öffnungen und/oder Ausschnitten einer Druckschablone frei von Gewebe sein.

Gleichzeitig besteht die Möglichkeit, dass innerhalb einer Öffnung und/oder eines Ausschnitts einer Druckschablone ein Formelement angeordnet ist, um die Form der Öffnung und/oder des Ausschnitts entsprechend der jeweils gewünschten Druckform zu definieren. Derartige Formelemente können mit einzelnen Haltefäden oder dergleichen an der gewünschten Position innerhalb der jeweiligen Öffnung und/oder des Ausschnitts gehalten werden.

Druckschablonen eignen sich insbesondere für den Druck von Lagen mit einer Dicke ab 100 µm, insbesondere ab 300 µm, besonders bevorzugt ab 500 µm. Der Druck mittels Druckschablone kann in verhältnismäßig hoher Geschwindigkeit erfolgen, da größere Auftragsstärken zu bewerkstelligen sind.

Gemäß einer weiteren vorteilhaften Ausgestaltung kann eine Trägerstruktur und/oder Unterlage für die Anordnung und/oder Aufnahme einer Mehrzahl voneinander getrennter Zellkulturen und/oder Zellagglomeration und/oder Gewebestrukturen ausgebildet sein. Eine solche Trägerstruktur kann insbesondere vordefinierte Anordnungs- und/oder Aufnahmeabschnitte für voneinander getrennte Zellkulturen und/oder Zellagglomeration und/oder Gewebestrukturen aufweisen. Hierdurch kann eine sichere und definierte Anordnung der gedruckten Zellen beziehungsweise durch die Zellen gebildete Zellkulturen und/oder Zellagglomeration und/oder Gewebestrukturen auf der Trägerstruktur gewährleistet werden.

In bevorzugter Weise können die Anordnungs- und/oder Aufnahmeabschnitte in mehreren Zeilen und Reihen auf der Trägerstruktur und/oder der Unterlage vorgesehen sein. Insbesondere kann sich die Anzahl der Zeilen von der Anzahl an Reihen unterscheiden. Die äußere Form der Trägerstruktur und/oder Unterlage kann an die Anzahl der Zeilen und Reihen angepasst sein. Weiter bevorzugt kann die Trägerstruktur und/oder die Unterlage Vertiefungen zur Aufnahme des Druckmediums aufweisen und/oder die Anordnungs- und/oder Aufnahmeabschnitte können als Vertiefung ausgebildet sein.

Es besteht ferner die Möglichkeit, dass eine Trägerstruktur zusammen und/oder abwechselnd mit dem lebende Zellen enthaltenden Druckmedium gedruckt und dadurch bereitgestellt wird. Hierdurch kann ein hohes Maß an Fertigungsflexibilität sichergestellt werden. Die Positionierung und Ausrichtung einer fertigen Trägerstruktur unterhalb eines Drucksiebs und/oder unterhalb einer Druckschablone kann somit entfallen. Der Vorbereitungssaufwand für das Drucken kann somit verringert werden.

Ebenso ist es möglich, dass eine Trägerstruktur vor dem Drucken des lebende Zellen enthaltenden Druckmediums gedruckt und auf diese Weise bereitgestellt wird. Die vollständig gedruckte Trägerstruktur kann dann als Druckunterlage eingesetzt werden.

Gemäß einer weiter bevorzugten Ausgestaltung kann das Druckmedium durch ein Drucksieb auf die Unterlage mit oder ohne Scaffold, Matrize und/oder strukturelles Gerüst gedruckt werden. Ferner kann auf der Unterlage wenigstens ein strukturelles Gerüst, eine Matrize und/oder ein Scaffold vorgesehen sein. Es ist ferner möglich, dass strukturelle Gerüste, Matrizen und/oder Scaffolds gemeinsam mit dem Druckmedium gedruckt werden und/oder in diesem enthalten sind. Scaffolds, Matrizes und Gerüste eignen sich in besonders vorteilhafter Weise für die Erzeugung dreidimensionaler Gewebestrukturen. Zellen, Zellkulturen und/oder Zellagglomerationen können auf diese Weise mit nur geringem Aufwand und gleichzeitig reproduzierbar dreidimensional ausgebildet werden beziehungsweise zu dreidimensionalen Gewebestrukturen entwickelt werden.

Beim Drucken des lebende Zellen aufweisenden Druckmediums können in besonders vorteilhafter Weise Kavitäten, Aussparungen, linienförmige oder kanalförmige Strukturen vorgesehen werden. Derartige Formen erlauben eine verbesserte Funktionalisierung der mit dem Druckmedium erzeugten Zellkulturen, Zellagglomerationen und/oder Geweben. Es können durch solche Kavitäten, Aussparungen und/oder Strukturen beispielsweise Scaffolds und/oder Blutgefäße und/oder Nerven beziehungsweise Nervenbahnen ausgebildet werden.

In besonders bevorzugter Weise kann eine Kombination von Materialien gedruckt werde, insbesondere um Gerüste und/oder Scaffolds und/oder Matrizes mit Zellen in einem einzigen Druckprozess mit nur geringem Aufwand herzustellen.

Es kann weiter von Vorteil sein, wenn das Druckmedium lagenweise gedruckt und/oder schichtweise aufgebaut wird. Ebenso kann eine einzelne Drucklage und/oder Druckschicht in einem einzigen Druckvorgang erzeugt werden und/oder eine Drucklage kann eine geringere Dicke in Druckaufbaurichtung aufweisen als die Erstreckung der Drucklage quer zur Druckaufbaurichtung. Es lassen sich auf diese Weise mit nur geringem Aufwand und gleichzeitig hoher Genauigkeit dreidimensionale Gewebestrukturen erzeugen.

Gemäß einer weiter vorteilhaften Ausgestaltung können durch den Druck des Druckmediums ein- oder mehrschichtige Zellagglomerationen und/oder ein- oder mehrschichtige Zellkulturen und/oder ein- oder mehrschichtige Gewebe und/oder Gewebestrukturen erzeugt werden. Der Druck einer einschichtigen Zellagglomeration und/oder Zellkultur und/oder Gewebestruktur kann mit nur geringem Aufwand und damit zu geringen Kosten realisiert werden. Mehrschichtige Zellagglomerationen und/oder Zellkulturen und/oder Gewebestrukturen ermöglichen besonders aussagekräftige Untersuchungen und/oder besonders weitreichende medizinische Behandlungen. Die Bedingungen lebender Organismen beziehungsweise die Eigenschaften menschlicher oder tierischer Gewebe- und/oder Organstrukturen lassen sich durch eine mehrschichtige Ausbildung besonders gut nachbilden.

In weiter bevorzugter Weise kann durch mehrmaliges Drucken eines lebende Zellen enthaltenden Druckmediums und/oder durch Variation der Zellen beziehungsweise des Zellen aufweisenden Druckmediums in einer Lagenaufbaurichtung (z-Richtung) einfach und schnell ein mehrlagiges Gewebe oder eine mehrlagige Gewebestruktur erzeugt werden. Bei einem solchen mehrlagigen Gewebe kann es sich beispielsweise um Haut oder auch um andere Zellgewebearten und/oder Organstrukturen handeln.

In weiter bevorzugter Weise kann das Druckmedium durch mindestens einen Rakelvorgang und/oder mindestens eine Druckrakelbewegung, insbesondere durch eine Mehrzahl von Rakelvorgängen und/oder eine Mehrzahl von Druckrakelbewegungen, durch das Drucksieb und/oder durch die Druckschablone hindurch und/oder auf die Unterlage befördert werden. Rakelvorgänge beziehungsweise Druckrakelbewegungen gestatten ein zügiges und gleichmäßigen Drucken des Druckmediums durch das Drucksieb beziehungsweise durch die Druckschablone hindurch. Insbesondere können auf diese Weise besonders reproduzierbare Ergebnisse erzielt werden.

In besonders bevorzugter Weise kann das Druckmedium durch mindestens einen Siebdruckvorgang, insbesondere durch eine Mehrzahl von Siebdruckvorgängen, gedruckt werden. Das Drucken des Druckmediums kann insbesondere im Siebdruckverfahren und/oder im Schablonendruckverfahren erfolgen, besonders bevorzugt im 2D Siebdruckverfahren oder 3D-Siebdruckverfahren. Hierdurch kann ein besonders hohes Maß an Produktivität und Genauigkeit gewährleistet werden. Im Siebdruckverfahren können verhältnismäßig große Volumina eines Druckmediums mit nur geringem Zeit- und Kostenaufwand gedruckt werden. Die Anordnung und/oder Erzeugung von Zellkulturen und/oder Zellagglomerationen und/oder Gewebe oder Gewebestrukturen kann somit schnell und kostengünstig erfolgen.

Gleichzeitig gewährleistet der Einsatz eines Siebdruckverfahrens, insbesondere eines 2D Siebdruckverfahrens oder 3D-Siebdruckverfahrens, eine schonende Verarbeitung des lebende Zellen enthaltenden Druckmediums. Die jeweiligen Zellen können im Siebdruckverfahren mit einer nur geringen Gefahr der Schädigung durch Scherbelastungen und/oder Druckbelastungen und/oder Reibbelastungen verarbeitet und/oder gedruckt werden. Für das Drucken des Druckmediums kann insbesondere eine 3D-Siebdruckanlage eingesetzt werden.

In weiter bevorzugter Weise werden Substrukturen der Zellkulturen und/oder Zellagglomerationen und/oder Gewebe und/oder Gewebestrukturen bis auf 100 Zellen genau gedruckt und/oder erzeugt, bevorzugt bis auf 50 Zellen, insbesondere 20 Zellen, weiter bevorzugt 10 Zellen, insbesondere bis auf eine einzelne Zelle genau gedruckt und/oder erzeugt. Derartige Druck- beziehungsweise Herstellgenauigkeiten können sowohl in Lagenaufbaurichtung (z-Richtung) und/oder in Richtungen quer zur Lageaufbaurichtung (x/y-Richtungen) erzielt werden. Insbesondere können derartige Genauigkeiten im Wege des 3D-Siebdruckens realisiert werden.

Der Siebdruckvorgang beziehungsweise die Mehrzahl von Siebdruckvorgängen kann in vorteilhafter Weise auf, insbesondere unmittelbar auf, die Unterlage erfolgen. Das Siebdrucken auf eine Unterlage, beispielsweise auf eine maschinenseitige Unterlage und/oder auf einen Zellträger oder Objektträger, ermöglicht ein hohes Maß an Reproduzierbarkeit sowie Handhabungsfreundlichkeit bei der Entnahme des gedruckten Objektes nach Abschluss des Druckvorgangs.

In noch weiter bevorzugter Ausgestaltung kann durch das Bedrucken der Unterlage mit dem Druckmedium eine dreidimensionale Zellkultur und/oder eine dreidimensionale Zellagglomeration erzeugt werden. Nach dem Bedrucken der Unterlage kann sich die jeweilige Zellkultur und/oder Zellagglomeration zu einer dreidimensionalen Gewebestruktur entwickelt. Ebenso kann sich die dreidimensionale Gewebestruktur bereits durch den Druck des Druckmediums entwickeln. Dementsprechend kann zwischen dem Abschluss des Druckens und der Entwicklung einer Zellkultur, Zellagglomeration und/oder Gewebestruktur eine Entwicklungszeit erforderlich sein. Über die Dauer einer solchen Entwicklungszeit können die Eigenschaften und/oder Beschaffenheiten der Gewebestruktur, Zellkulturen beziehungsweise Zellagglomerationen beeinflusst werden. Dreidimensionale Zellkulturen und/oder Zellagglomerationen und/oder Gewebestrukturen lassen sich insbesondere durch mehrmaliges Drucken beziehungsweise durch 3D-Siebdruck erzeugen.

Gemäß einer weiter bevorzugten Ausgestaltung kann vorgesehen sein, dass sich die gedruckten Zellen und/oder Zellagglomerationen durch den Druck oder nach dem Bedrucken zu Organgewebe, insbesondere Lebergewebe, Nierengewebe, Herzgewebe, Hautgewebe und/oder Muskelgewebe, und/oder zu einem teilweisen oder vollständigen Organ, insbesondere Leber, Niere, Herz und/oder Haut, bevorzugt zur Transplantation, entwickeln. Solche Gewebestrukturen eignen sich in besonders vorteilhafter Weise für medizinische Behandlungen und/oder pharmakologische Untersuchungen.

In weiter bevorzugter Weise können sich die gedruckten Zellen durch den Druck und/oder nach dem Druck zu lebensmitteltauglichem Gewebe und/oder zu einem Lebensmittel entwickeln. Die jeweilige Zellkultur und/oder die Zellagglomeration kann sich durch den Druck und/oder nach dem Druck insbesondere zu synthetischem Fleisch entwickeln, das zum Verzehr geeignet sein kann. Insbesondere ist es möglich, dass sich die gedruckten Zellen und/oder Zellagglomerationen durch den Druck oder nach dem Bedrucken zu einem fleischhaltigem Lebensmittel entwickeln.

In weiter bevorzugter Weise kann das Drucksieb und/oder die Druckschablone wenigstens einen Ausschnitt, insbesondere mehrere Ausschnitte, für den Durchlass des Druckmediums aufweisen, insbesondere getrennt voneinander ausgebildete Ausschnitte. Es lässt sich damit ein definierter Durchtritt des Druckmediums durch das Drucksieb und/oder die Druckschablone realisieren. Insbesondere kann das Druckmedium hierdurch in definierter Weise an unterschiedlichen und/oder voneinander beabstandeten Positionen gedruckt und/oder auf die Unterlage beziehungsweise zuvor gedruckte Drucklagen aufgebracht werden. Ferner kann das Drucksieb wenigstens einen Ausschnitt mit ineinander übergehenden Abschnitten aufweisen.

In weiter bevorzugter Weise können die Ausschnitte des Drucksiebs und/oder der Druckschablone mit vordefinierten Anordnungs- und/oder Aufnahmeabschnitten der Unterlage korrespondieren. Hierdurch kann das Druckmedium gezielt in und/oder auf die Anordnungs- und/oder Aufnahmeabschnitte der Unterlage gedruckt werden. Insbesondere kann durch eine solche Ausgestaltung vermieden werden, dass ein versehentliches oder unerwünschtes Bedrucken der Trägerstruktur außerhalb der Anordnungs- und/oder Aufnahmeabschnitte erfolgt. Gleichzeitig kann hierdurch ein hohes Druckvolumen pro Druckvorgang realisiert werden.

Gemäß einer weiter bevorzugten Ausgestaltung kann zumindest ein Ausschnitt des Drucksiebs und/oder der Druckschablone für den Durchlass des Druckmediums einen Durchmesser von 1 mm bis 100 mm aufweisen, insbesondere von 2 mm bis 50 mm, bevorzugt von 2 mm bis 40 mm, bevorzugt von 2 mm bis 30 mm, weiter bevorzugt von 3 mm bis 30 mm, weiter bevorzugt von 3 mm bis 25 mm, weiter bevorzugt von 3 mm bis 20 mm, noch weiter bevorzugt von 4 mm bis 20 mm, weiter bevorzugt von 4 mm bis 15 mm, weiter bevorzugt von 4 mm bis 10 mm, noch weiter bevorzugt von 4 mm bis 9 mm, weiter bevorzugt von 4 mm bis 8 mm, noch weiter bevorzugt von 5 mm bis 10 mm, weiter bevorzugt von 5 mm bis 8 mm. Ebenso kann zumindest ein Ausschnitt des Drucksiebs und/oder der Druckschablone für den Durchlass des Druckmediums eine entsprechend der voranstehenden Durchmesserwerte korrespondierende Größe aufweisen. Dies gilt insbesondere für Ausschnittformen, die von einer kreisrunden Form abweichen. Durch einen derart bemessenen Ausschnitt im Drucksieb und/oder in der Druckschablone kann einerseits eine nur geringe mechanische Belastung der lebenden Zellen im Druckmedium und gleichzeitig auch ein verhältnismäßig großes Druckvolumen je Druckvorgang sichergestellt werden.

Gemäß einer bevorzugten Ausgestaltung kann eine Mehrzahl von Oberflächen-, Anordnungs- und/oder Aufnahmeabschnitten der Unterlage zeitgleich und/oder in einem Druckvorgang und/oder durch einen Rakelvorgang mit dem Druckmedium bedruckt und/oder befüllt werden. Ebenso können unterschiedliche Abschnitte einer zuvor gedruckten Lage zeitgleich und/oder in einem Druckvorgang und/oder durch einen Rakelvorgang mit dem Druckmedium bedruckt werden. Dies kann mit nur geringem Aufwand bewerkstelligt werden. Insbesondere können in einem Druckvorgang sämtliche Anordnungs- und/oder Aufnahmeabschnitte der Unterlage oder sämtliche Abschnitte der zuvor gedruckten Lage mit dem Druckmedium bedruckt werden. Das sequentielle Bedrucken einzelner Abschnitte in vielen Einzelschritten kann somit vermieden werden.

Es ist weiterhin möglich, dass unterschiedliche Oberflächen-, Anordnungs- und/oder Aufnahmeabschnitte der Unterlage oder unterschiedliche Abschnitte einer zuvor gedruckten Lage in zeitlicher Abfolge entsprechend ihres Abstands zueinander sowie entsprechend der jeweiligen Rakelgeschwindigkeit bedruckt werden. Die Einzelnen Anordnungs- und/oder Aufnahmeabschnitte der Unterlage können somit entsprechend einer Rakelbewegung bedruckt werden. Eine unmittelbar zeitgleiches Bedrucken der Anordnungs- und/oder Aufnahmeabschnitte der Unterlage oder der Abschnitte einer zuvor gedruckten Lage kann somit unterbleiben, wodurch die Verfahrensflexibilität erhöht wird.

Gemäß einer weiter bevorzugten Ausführungsform des erfindungsgemäßen Verfahrens kann das Druckmedium an vereinzelten Positionen auf eine Trägerstruktur gedruckt werden. Durch das Bedrucken einer Trägerstruktur mit dem Druckmedium können in besonders vorteilhafter Weise voneinander unabhängige und/oder fluidtechnisch getrennte Druckabschnitte erzeugt werden. In der Folge lassen sich mehrere voneinander unabhängige Gewebe, Gewebestrukturen, Zellkulturen und/oder Zellagglomerationen erzeugen, die für eine verhältnismäßig große Anzahl an medizinischen Behandlungen und/oder pharmakologischen Untersuchungen zur Verfügung stehen.

Gemäß einer weiter bevorzugten Ausgestaltung können zum Drucken eines Gewebes unterschiedliche Drucksiebe eingesetzt werden, insbesondere um unterschiedlich geformte Drucklagen zu erzeugen. Ferner können durch die unterschiedliche Formgebung verschiedener Drucksiebe komplexe dreidimensionale Gewebestrukturen erzeugt werden, insbesondere Blutgefäße, Gefäßwände, Kanäle, strukturelle Gerüste, Matrizen und/oder Scaffolds. Es kann hierdurch eine besonders vorteilhafte Nachbildung von biologischen Gewebestrukturen realisiert werden.

In weiter bevorzugter Weise können zum Drucken unterschiedlicher Lagen unterschiedliche Druckmedien und/oder Druckmedien mit unterschiedlichen Zellen verwendet werden, insbesondere zur Erzeugung von Gewebe mit unterschiedlichen Lageneigenschaften und/oder zur Erzeugung von unterschiedlichen Hautlagen. Weiterhin kann in einer Druckaufbaurichtung (z-Richtung) eine Variation des Druckmediums vorgenommen werden. Es können auf diese Weise Gewebestrukturen mit einem hohen Maß an Funktionalität erzeugt werden.

Es kann ebenso von Vorteil sein, wenn innerhalb einer Drucklage, insbesondere entlang einer x/y-Richtung und/oder quer zu einer Druckaufbaurichtung, eine Gewebevariation erzeugt wird. Ferner können Gewebevariationen durch den Einsatz verschiedener Drucksiebe und/oder Druckschablonen erzeugt werden, insbesondere verschiedene Drucksiebe und/oder Druckschablonen pro Zelltyp und/oder Druckbereich. Hierdurch können besonders detaillierte Nachbildungen von biologischen Gewebestrukturen erzeugt werden.

Es kann weiter von Vorteil sein, während des Druckens, vor dem Drucken und/oder nach dem Drucken eine Überlagerung des Druckmediums mit einem sterilem Gasmedium vorzunehmen, insbesondere mit steriler Luft. Sterilitätsanforderungen an den Umgang mit und/oder die Verarbeitung von lebenden Zellen können auf diese Weise sicher befolgt werden. Eine unerwünschte Verunreinigung der Zellkulturen und/oder Zellagglomerationen und/oder des gedruckten und/oder erzeugten Gewebes kann somit vermieden werden.

Bei dem Druckmedium kann es sich insbesondere um ein Nährmedium und/oder ein Nährstoffe enthaltendes Medium, insbesondere eine Nährflüssigkeit, handeln. Eine solches Druckmedium beziehungsweise Nährmedium kann insbesondere als sogenannte Bio-Ink ausgebildet sein.

Gemäß einer noch weiteren bevorzugten Ausgestaltung kann das Druckmedium als Druckpaste und/oder niederviskose oder mittelviskose Nährflüssigkeit und/oder flüssige Suspension ausgebildet sein. In rein gel-förmigen Medien können Zellen und/oder Nährstoffe nur in eingeschränkter Weise diffundieren, was dazu führen kann, dass die Verfügbarkeit der Nährstoffe eingeschränkt ist und somit auch die Lebensdauer der Zellen. Durch die Ausbildung des Druckmedium als verhältnismäßig dünnflüssige Druckpaste und/oder niederviskose oder mittelviskose Nährflüssigkeit und/oder flüssige Suspension kann ein hohes Maß an Nährstoffversorgung der lebenden Zellen innerhalb des Druckmediums sichergestellt werden.

Ebenso ist es möglich, dass das Druckmedium gelförmig und/oder als hochviskoses Nährmedium ausgebildet ist, wodurch die Verarbeitung beziehungsweise das Drucken vereinfacht werden kann.

Ferner kann das Druckmedium als Sol-Gel Matrix ausgebildet sein und/oder eine variable Viskosität aufweisen und/oder die Viskosität des Druckmediums kann durch einen Trocknungsschritt und/oder Temperierungsschritt, insbesondere einer Temperaturverringerung oder Temperaturerhöhung, veränderbar sein. Je nach erfolgtem Verarbeitungsschritt kann die Viskosität damit verändert werden. Es kann hierdurch über verhältnismäßig lange Zeiträume ein hohes Maß an Nährstoffversorgung gewährleistet werden und gleichzeitig kann die Viskosität zwecks Verarbeitung oder Weiterverarbeitungsschritten verändert werden.

Das Druckmedium kann weiterhin strukturviskos und/oder scherverdünnend ausgebildet sein. Bei verhältnismäßig hohen Scherbelastungen des Druckmediums kann sich hierdurch eine temporär geringere Viskosität einstellen und dadurch die Scherbelastung verringert werden. Die Gefahr von Zellbeschädigungen wird dadurch weiter verringert.

Erfindungsgemäß erfolgt zwischen aufeinanderfolgenden Druckschritten ein Trocknungs- und/oder Temperierungsschritt des aufgedruckten Druckmediums. Es ist ebenso möglich, dass nach dem Drucken des Druckmediums ein Sol-Gel-Übergang erzeugt wird, insbesondere durch einen Trocknungs- und/oder Temperierungsschritt, und/oder dass nach einem erzeugten Sol-Gel-Übergang ein weiterer Druckvorgang vorgenommen wird. Die Eigenschaften des Druckmediums beziehungsweise der dadurch erzeugten Zellkultur und/oder Zellagglomeration lassen sich hierdurch gezielt in Abhängigkeit des jeweils erfolgten Verfahrensstadiums beeinflussen.

In weiter bevorzugter Weise kann das Druckmedium beim Drucken flüssig sein oder eine geringe Viskosität aufweisen, sodass ein einfaches Drucken möglich ist. Im Anschluss an den Druck beziehungsweise den Druck einer Drucklage kann die jeweilige Drucklage verfestigt beziehungsweise die Viskosität des gedruckten Mediums erhöht werden, beispielsweise durch Temperierung und/oder durch Erzeugung eines Sol-Gel Übergangs. Das jeweilige Druckobjekt beziehungsweise die durch Drucken erzeugte Zellkultur und/oder Zellagglomeration kann hierdurch in geeigneter Weise in einer Lagenaufbaurichtung (z-Richtung) aufgebaut werden.

Sobald das jeweilige Druckobjekt in der Höhe (z-Richtung) fertig aufgebaut ist, kann dieses in eine geeignete Form und/oder Schablone eingesetzt werden oder die Form und/oder Schablone kann auf das Druckobjekt aufgesetzt werden, um dieses zu umgeben. Das Druckobjekt kann somit in x/y- Richtung begrenzt werden. Das gedruckte Medium beziehungsweise die durch Drucken erzeugte Zellkultur und/oder Zellagglomeration kann wieder in einen flüssigeren Zustand beziehungsweise in einen Zustand mit geringerer Viskosität gebracht werden, ohne dass es zu einem zerlaufen kommt. Hierdurch können Nährstoffe innerhalb der durch Drucken erzeugten Zellkultur und/oder Zellagglomeration wieder frei zirkulieren. Gewebe kann sich in verbesserter Weise entwickeln.

In weiter bevorzugter Ausgestaltung kann das Druckmedium teilungsfähige und/oder zur Teilungsfähigkeit induzierbare Zellen enthalten. Ferner kann es sich bei den lebenden Zellen des Druckmediums um menschliche, tierische und/oder pflanzliche Zellen handeln. Insbesondere kann das Druckmedium alle Arten von menschlichen, tierischen und/oder pflanzlichen Zellen enthalten. Besonders bevorzugt kann es sich bei den lebenden Zellen um alle teilungsfähigen oder zur Teilungsfähigkeit induzierbaren Zellen des menschlichen oder tierischen Körpers oder von Pflanzen handeln. Es ergibt sich hierdurch eine besondere Eignung für medizinische und pharmakologische Untersuchungen.

In weiter bevorzugter Weise kann das Druckmedium lebende Zellen aus der Gruppe der Primärzellen aufweisen, insbesondere aller Arten von menschlichen, tierischen und/oder pflanzlichen Primärzellen.

Ebenso kann das Druckmedium lebende Zellen aus der Gruppe der Zelllinien aufweisen, insbesondere aller Arten von menschlichen, tierischen und/oder pflanzlichen etablierten Zelllinien.

Bei Primärzellen kann es sich beispielsweise handeln um Organzellen, insbesondere Organzellen aller Organe, Hautzellen, Fibroblasten, Chondroblasten, Osteoblasten, Muskelzellen, Herzmuskelzellen, Nervenzellen, Leberzellen, Inselzellen, vaskuläre Zellen, Drüsengewebezellen, Tumorzellen, insbesondere Tumorzellen aller Tumorgewebe, Stammzellen, insbesondere hämatopoetische, mesenchymale und/oder neuronale Stammzellen und/oder induzierte pluripotente Stammzellen aus Geweben, wie beispielsweise Fettgewebe, Haut und/oder Nabelschnur, und/oder totipotente Stammzellen, insbesondere Eizellen und/oder Embryonalzellen, und/oder pluripotente, multipotente, oligopotente und/oder unipotente Stammzellen, Blutzellen und/oder Immunzellen.

Bei Zelllinien kann es sich beispielsweise um immortalisierte Zellen der voranstehend benannten Zelltypen und/oder Zellen der voranstehend genannten Gewebetypen handeln.

Es kann weiter von Vorteil sein, wenn das Druckmedium und/oder das erzeugte Gewebe unterschiedliche Zellen und/oder Zellentypen enthält, insbesondere unterschiedliche der voranstehend benannten Zellen und/oder Zellentypen. Es kann sich hierdurch eine weiter verbesserte Eignung der erzeugten Zellkulturen und/oder Zellagglomerationen und/oder Gewebestrukturen für medizinische und/oder pharmakologische Untersuchungen und/oder Behandlungen ergeben.

Weiter bevorzugt kann ein aus dem Druckmedium erzeugtes Gewebe unterschiedliche Zellen und/oder Zellentypen enthalten, insbesondere unterschiedliche der voranstehend benannten Zellen und/oder Zellentypen. Es kann sich hierdurch ebenfalls eine weiter verbesserte Eignung des erzeugten Gewebes für medizinische und/oder pharmakologische Untersuchungen ergeben.

Es kann weiter von Vorteil sein, wenn das Druckmedium zumindest einen Wachstumsfaktor und/oder zumindest ein Protein und/oder extrazelluläres Matrixprotein enthält, insbesondere ein Wachstumsfaktor und/oder Protein aus der Gruppe der Zytokine, insbesondere als Wachstumsregulatoren, Interferone und/oder Interleukine, Membranbestandteile, Laminine, Kollagene, insbesondere Kollagen Typ 4, Proteoglykane, Entactine, Nidogene, Zelladhärenzfaktoren, insbesondere Fibronectin und/oder Vitronectin, Wachstumsfaktoren, insbesondere der EGF-Familie, der TGF-Familie, PDGF, VEGF, Somatomedine, insbesondere IGF, NGF, PTGF, und/oder Schutzfaktoren, insbesondere gewebespezifische Plasminogenaktivatoren, Serumalbumine und/oder CMC. In besonderes bevorzugter Weise kann das Druckmedium zumindest eine oder mehrere dieser Substanzen aus den vorstehend benannten Gruppen aufweisen. Es kann sich hierdurch eine weiter verbesserte Eignung der mittels des Druckmediums erzeugten Zellkulturen, Zellagglomerationen und/oder Gewebestrukturen für die Durchführung von medizinischen und/oder pharmakologischen Untersuchungen und/oder Behandlungen ergeben.

In weiter bevorzugter Weise kann das Druckmedium Zellen mit einer Größe von 5-50µm, 5-40µm, 10-50µm, 10-40µm, 10-30µm, 20-40µm, 25-40µm, 25-30µm im Durchmesser oder in der Querschnittslänge aufweisen. Eine Zellkultur und/oder eine Zellagglomeration und/oder ein daraus erzeugtes Gewebe kann eine Anzahl von wenigstens 5 Zellen, insbesondere von wenigstens 10 Zellen, bevorzugt von wenigstens 20 Zellen, insbesondere wenigstens 50 Zellen, weiter bevorzugt von wenigstens 100 Zellen, weiter bevorzugt von wenigstens 200 Zellen oder wenigstens 300 Zellen, aufweisen. Ferner kann eine Zellkultur und/oder eine Zellagglomeration und/oder ein daraus erzeugtes Gewebe eine Anzahl von bis zu 100.000.000 Zellen, von bis zu 10.000.000 Zellen, von bis zu 1.000.000 Zellen, von bis zu 100.000 Zellen, von bis zu 10.000 Zellen, von bis zu 1000 Zellen aufweisen, insbesondere von bis zu 500 Zellen. Insbesondere kann eine Zellkultur und/oder eine Zellagglomeration und/oder ein daraus erzeugtes Gewebe eine Anzahl von 5 bis 100.000.000 Zellen, von 5 bis 10.000.000 Zellen, 5 bis 1.000.000 Zellen, von 5 bis 100.000 Zellen, von 5 bis 10.000 Zellen, von 5 bis 1000 Zellen aufweisen, insbesondere von 10 bis 1000 Zellen, 20 bis 500 Zellen, 50 bis 500 Zellen, 100 bis 500 Zellen, 200 bis 500 Zellen oder 300 bis 500 Zellen. Hierdurch können besonders aussagekräftige Untersuchungen mittels der durch das Druckmedium erzeugten Zellkulturen und/oder Zellagglomerationen vorgenommen oder für medizinische Behandlungen besonders geeignete Gewebestrukturen hergestellt werden.

Ein weiterer unabhängiger Aspekt der vorliegenden Erfindung betrifft ein Verfahren zur Erzeugung von biologischem Gewebe, bei dem eine Unterlage für Zellgewebe bereitgestellt wird, bei dem ein lebende Zellen enthaltenes Druckmedium mittels Siebdrucks auf die Unterlage gedruckt wird und bei dem sich die gedruckten Zellen durch den Druck und/oder nach dem Druck zu Gewebe entwickeln.

Ein weiterer unabhängiger Aspekt der vorliegenden Lehre betrifft ein Gewebe und/oder eine Gewebestruktur, insbesondere ein Organ, das durch ein voranstehend beschriebenes Verfahren erzeugt ist.

Gemäß einer besonders bevorzugten Ausgestaltung können die Zellenkulturen und/oder Zellagglomerationen und/oder Gewebestrukturen und/oder Gewebeabschnitte hinsichtlich des metabolischen Zustands der Zellen, des Zellalter und/oder der Zellenanzahl und/oder der Dimension der Zellenanzahl gleich sein. Ebenso ist es möglich, dass bei den Zellenkulturen und/oder Zellagglomerationen und/oder Gewebestrukturen und/oder Gewebeabschnitten Abweichungen hinsichtlich des metabolischen Zustands der Zellen, des Zellalters und/oder Zellenanzahl geringer sind als 20%, insbesondere geringer als 10%, geringer als 5%, geringer als 3%, geringer als 2% oder geringer als 1%. Durch eine derartige Ausgestaltung können die Zellenkulturen und/oder Zellagglomerationen und/oder Gewebestrukturen beziehungsweise Gewebeabschnitte für besonders weitreichende medizinische Behandlungen eingesetzt werden.

Weitere Ausgestaltungen der vorliegenden Erfindung ergeben sich aus Kombinationen der in den Ansprüchen, der Beschreibung sowie den Figuren offenbarten Merkmale. Die vorliegende Erfindung wird nachfolgend anhand von Ausführungsbeispielen und zugehörigen Zeichnungen erläutert.

Es zeigen jeweils schematisch:
- Fig. 1: eine Unterlage für Zellgewebe und ein darüber angeordnetes Druck-sieb beziehungsweise eine Druckschablone in Perspektivdarstellung,
- Fig. 2: zwei durch ein erfindungsgemäßes Verfahren erzeugte Gewebe auf einer Unterlage gemäß Fig. 1 in Perspektivdarstellung.

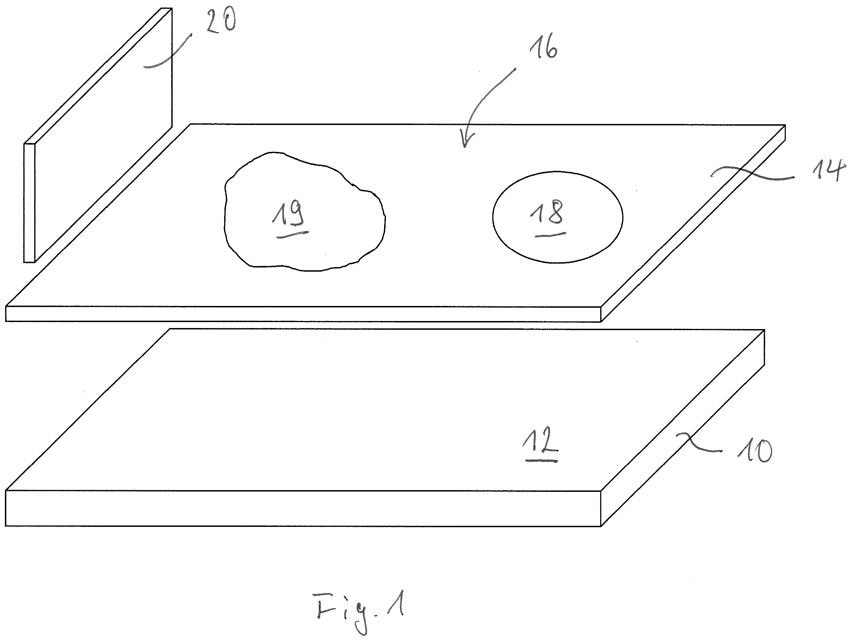

In Fig. 1 ist eine Unterlage 10 für Zellgewebe, Zellkulturen und/oder Zellagglomerationen in Perspektivdarstellung gezeigt. Bei der Unterlage 10 kann es sich insbesondere um eine maschinenseitige Unterlage handeln, auf die ein Druckmedium gedruckt werden kann. Eine solche maschinenseitige Unterlage 10 kann fest mit einer hier nicht gezeigten Druckanlage, insbesondere einer 3D-Siebdruckanlage, verbunden sein. Ebenso kann es sich bei der Unterlage 10 um eine Trägerstruktur, eine Zellkulturplatte, einen Objektträger beziehungsweise um ein sogenanntes Array für Zellgewebe, Zellkulturen und/oder Zellagglomerationen handeln. Eine solche Unterlage kann für den jeweiligen Druckvorgang oder Druckprozess in einer Druckanlage positioniert und anschließend wieder aus dieser entnommen werden.

Die Unterlage 10 kann zur Positionierung, Aufnahme und/oder Entwicklung von hier nicht näher dargestellten Zellgeweben, Zellkulturen und/oder Zellagglomerationen geeignet oder ausgebildet sein. Insbesondere kann ein hier nicht näher dargestelltes lebende Zellen enthaltendes Druckmedium durch Drucken auf eine Oberfläche 12 der Unterlage 10 befördert werden, was nachstehend näher erläutert wird. Sobald ein solches Druckmedium auf der Oberfläche 12 angeordnet ist, kann sich dieses zu Zellgewebe, Zellkulturen und/oder Zellagglomerationen entwickeln. Ebenso kann ein bereits aus Zellkulturen und/oder Zellagglomerationen bestehendes und/oder Zellkulturen und/oder Zellagglomerationen enthaltendes Druckmedium durch Drucken auf der Oberfläche 12 angeordnet und dadurch zu Gewebe entwickelt werden. Demnach kann sich das Druckmedium durch den Druck und/oder nach dem Druck zu Zellgewebe entwickeln.

In der Fig. 1 ist ferner eine Druckschablone 14 in schematischer Perspektivdarstellung gezeigt. Die Druckschablone 14 kann durch eine Platte 16 gebildet sein. Anstelle der Druckschablone 14 kann auch ein hier nicht gezeigtes Drucksieb vorgesehen sein.. Die Druckschablone 14 kann zumindest einen Ausschnitt 18, 19 oder auch eine Mehrzahl von Ausschnitten 18, 19 für den Durchlass eines Druckmediums aufweisen. Im Falle mehrerer Ausschnitte 18, 19 können diese insbesondere getrennt voneinander ausgebildet sein. Der Ausschnitt 18 kann beispielsweise einen Durchmesser von 1 mm bis 100 mm, insbesondere von 2 mm bis 50 mm, bevorzugt von 2 mm bis 40 mm, bevorzugt von 2 mm bis 30 mm, weiter bevorzugt von 3 mm bis 30 mm, weiter bevorzugt von 3 mm bis 25 mm, weiter bevorzugt von 3 mm bis 20 mm, noch weiter bevorzugt von 4 mm bis 20 mm, weiter bevorzugt von 4 mm bis 15 mm, weiter bevorzugt von 4 mm bis 10 mm, noch weiter bevorzugt von 4 mm bis 9 mm, weiter bevorzugt von 4 mm bis 8 mm, noch weiter bevorzugt von 5 mm bis 10 mm, weiter bevorzugt von 5 mm bis 8 mm, aufweisen.

Der Ausschnitt 19 kann eine Größe aufweisen, die mit den voranstehend benannten durchmesserbasierten Größenangaben korrespondiert. Die Ausschnitte 18 und 19 können unterschiedliche Durchmesser und/oder Größen oder auch identische Durchmesser und/oder Größen aufweisen.

Gemäß der vorliegenden Erfindung wird ein lebende Zellen enthaltenes Druckmedium durch ein Drucksieb und/oder durch die Druckschablone 14 gedruckt.

Für das Drucken des Druckmediums kann ein Drucksieb und/oder die Druckschablone 14 oberhalb der Unterlage 10 angeordnet werden, wie in Fig. 1 gezeigt. Das ferner in Fig. 1 schematisch gezeigte Druckrakel 20 kann für das Drucken eines Druckmediums durch ein Drucksieb und/oder durch die Druckschablone 16 eingesetzt werden. Dabei kann das Druckmedium durch mindestens einen Rakelvorgang und/oder mindestens eine Druckrakelbewegung, insbesondere durch eine Mehrzahl von Rakelvorgängen des Druckrakels 20 und/oder eine Mehrzahl von Druckrakelbewegungen des Druckrakels 20, durch ein Drucksieb und/oder die Druckschablone 16 hindurch gedruckt werden. Hierdurch lässt sich das Druckmedium besonders zuverlässig auf die Unterlage 10 befördern.

Das Drucken des Druckmediums mittels eines Drucksiebs und/oder mittels der Druckschablone 14 und/oder des Druckrakels 20 kann insbesondere im Siebdruckverfahren erfolgen. Es kann sich bei dem Siebdruckverfahren insbesondere um ein 2D-Siebdruckverfahren oder 3D-Siebdruckverfahren handeln.

Es besteht ferner die Möglichkeit, dass die Unterlage 10, insbesondere bei Ausbildung als Trägerstruktur, zusammen und/oder abwechselnd mit dem lebende Zellen enthaltenden Druckmedium gedruckt wird. Insbesondere kann das Drucken des lebende Zellen enthaltenden Druckmediums zusammen und/oder abwechselnd mit dem Drucken einer als Trägerstruktur ausgebildete Unterlage 10 im 3D-Siebdruckverfahren erfolgen. Ferner kann die Unterlage 10 vor dem Drucken des lebende Zellen enthaltenden Druckmediums gedruckt werden, insbesondere im 3D-Siebdruckverfahren. Ebenso kann eine als Trägerstruktur ausgebildete Unterlage 10 anderweitig hergestellt sein und vor dem Drucken des lebende Zellen enthaltenden Druckmediums bereitgestellt werden.

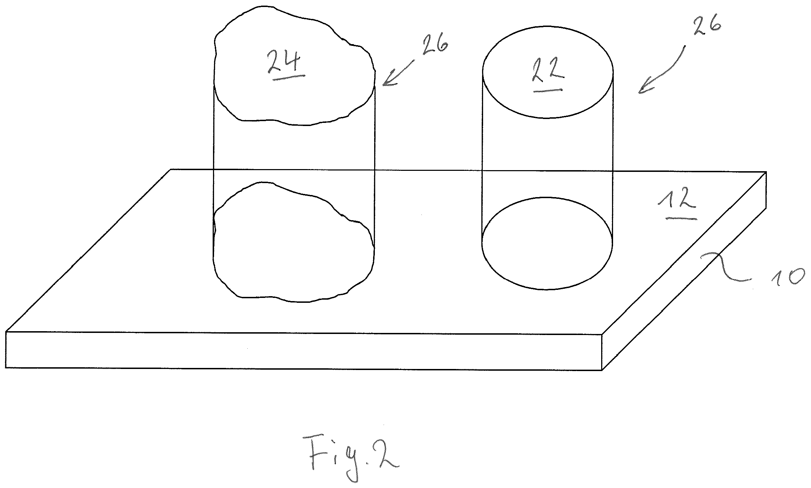

In Fig. 2 sind mehrere Gewebe 22 und 24 auf einer Unterlage 10 in Perspektivdarstellung gezeigt. Bei den Geweben 22 und 24 handelt es sich insbesondere um biologische Zellgewebe beziehungsweise Gewebestrukturen. Bei den Geweben 22 und 24 kann es sich um Teile oder Abschnitte eines Organs handeln, insbesondere um Teile oder Abschnitte eines menschlichen oder tierischen Organs. Ebenso kann auch ein vollständiges Organ gedruckt werden. Durch den Druck des Druckmediums können ein- oder mehrschichtige Gewebe 22 und 24 erzeugt werden, was hier nicht näher dargestellt ist. Ebenso können durch den Druck des Druckmediums ein- oder mehrschichtige und/oder ein- oder mehrschichtige Zellagglomerationen 24 erzeugt werden, die sich dann zu den Gewebe 22 und oder 24 entwickeln.

Ferner kann durch das Bedrucken der Trägerstruktur 10 mit dem Druckmedium eine dreidimensionale Zellkultur und/oder Zellagglomeration erzeugt werden. Nach dem Bedrucken der Unterlage 10 mit dem Druckmedium kann sich die jeweilige Zellkultur und/oder Zellagglomeration zu einem dreidimensionalen Gewebe 22, 24 entwickeln.

Es besteht ferner die Möglichkeit, dass in Druckabschnitten 26 beziehungsweise den Geweben 22, 24 strukturelle Gerüste und/oder Scaffolds vorgesehen sind, die hier ebenfalls nicht näher dargestellt sind. Solche strukturellen Gerüste und/oder Scaffolds können gemeinsam mit dem Druckmedium gedruckt werden und/oder in diesem enthalten sein.

In besonders bevorzugter Weise können mehrere Abschnitte der Oberfläche 12 der Unterlage 10 zeitgleich und/oder in einem Druckvorgang und/oder durch einen Rakelvorgang mit dem Druckmedium bedruckt und/oder befüllt werden. Ebenso können unterschiedliche Abschnitte der Oberfläche 12 der Unterlage 10 in zeitlicher Abfolge entsprechend ihres Abstands zueinander sowie entsprechend der jeweiligen Rakelgeschwindigkeit bedruckt und/oder befüllt werden.

Durch das Bedrucken der Unterlage 10 mit dem Druckmedium können voneinander unabhängige und/oder fluidtechnisch getrennte Druckabschnitte 26 erzeugt werden. Insbesondere kann je Oberflächenabschnitt ein Druckabschnitt 26 erzeugt werden. Ein Druckabschnitt 26 kann ein Gewebe 22, 24 und/oder eine Zellkultur und/oder eine Zellagglomeration bilden oder sich zu einer Zellkultur und/oder einer Zellagglomeration und/oder einem Gewebe 22, 24 entwickeln. Ferner können sich die Zellkulturen und/oder die Zellagglomerationen nach dem Drucken zu Gewebe entwickeln.

In der Anordnung gemäß Fig. 2 können die einzelnen Gewebe 22, 24 und/oder Zellkulturen und/oder Zellagglomerationen hinsichtlich des metabolischen Zustands der Zellen, des Zellalters und/oder der Zellenanzahl und/oder der Dimension der Zellenanzahl gleich sein. Ebenso besteht die Möglichkeit, dass in der Anordnung 11 gemäß Fig. 2 Abweichungen hinsichtlich des metabolischen Zustands der Zellen, des Zellalters und/oder der Zellenanzahl geringer als 20%, insbesondere geringer als 10%, geringer als 5%, geringer als 3%, geringer als 2% oder geringer als 1% sind.

Das Druckmedium beziehungsweise die damit erzeugten Gewebe 22, 24 und/oder Zellkulturen und/oder Zellagglomerationen können teilungsfähige und/oder zur Teilungsfähigkeit induzierbare Zellen enthalten. Es kann sich bei den lebenden Zellen des Druckmediums beziehungsweise der Gewebe 22, 24 und/oder Zellenkulturen und/oder Zellagglomerationen um menschliche, tierische und/oder pflanzliche Zellen handeln. Insbesondere kann/können das Druckmedium beziehungsweise die Gewebe 22, 24 und/oder Zellkulturen und/oder Zellagglomerationen alle Arten von menschlichen, tierischen und/oder pflanzlichen Zellen enthalten. Besonders bevorzugt kann es sich bei den lebenden Zellen des Druckmediums beziehungsweise der Gewebe 22, 24, der Zellenkulturen und/oder Zellagglomerationen um alle teilungsfähigen oder zur Teilungsfähigkeit induzierbaren Zellen des menschlichen oder tierischen Körpers oder von Pflanzen handeln.

Ferner kann das Druckmedium beziehungsweise ein daraus erzeugtes Gewebe 22, 24 und/oder eine aus dem Druckmedium erzeugte Zellkultur und/oder Zellagglomeration lebende Zellen aus der Gruppe der Primärzellen aufweisen, insbesondere alle Arten von menschlichen, tierischen und/oder pflanzlichen Primärzellen. Ebenso kann das Druckmedium beziehungsweise ein daraus erzeugtes Gewebe 22, 24 oder eine daraus erzeugte Zellkultur und/oder Zellagglomeration lebende Zellen aus der Gruppe der Zelllinien aufweisen, insbesondere alle Arten von menschlichen, tierischen und/oder pflanzlichen etablierten Zelllinien. Das Druckmedium beziehungsweise ein daraus erzeugtes Gewebe 22, 24 oder eine daraus erzeugte Zellkultur und/oder Zellagglomeration kann schließlich unterschiedliche Zellen und/oder Zellentypen enthalten.

Ferner kann das Druckmedium beziehungsweise ein daraus erzeugtes Gewebe 22, 24 oder eine daraus erzeugte Zellkultur und/oder Zellagglomeration zumindest einen Wachstumsfaktor und/oder zumindest ein Protein und/oder extrazelluläres Matrixprotein enthalten, insbesondere ein Wachstumsfaktor und/oder Protein aus der Gruppe der Zytokine, insbesondere als Wachstumsregulatoren, Interferone und/oder Interleukine, Membranbestandteile, Laminine, Kollagene, insbesondere Kollagen Typ 4, Proteoglykane, Entactine, Nidogene, Zelladhärenzfaktoren, insbesondere Fibronectin und/oder Vitronectin, Wachstumsfaktoren, insbesondere der EGF-Familie, der TGF-Familie, PDGF, VEGF, Somatomedine, insbesondere IGF, NGF, PTGF, und/oder Schutzfaktoren, insbesondere gewebespezifische Plasminogenaktivatoren, Serumalbumine und/oder CMC. Ebenso kann das Druckmedium beziehungsweise ein daraus erzeugtes Gewebe 22, 24 oder eine daraus erzeugte Zellkultur und/oder Zellagglomeration zumindest eine oder mehrere dieser Substanzen aus den vorstehend benannten Gruppen aufweisen.

Die gemäß voranstehend beschriebenen Verfahren erzeugten Gewebe 22, 24 oder Zellenkulturen und/oder Zellagglomerationen eignen sich besonders bevorzugt zur Durchführung von medizinischen Behandlungen und/oder pharmakologischen Untersuchungen.

## Patentansprüche

1. Verfahren zur Erzeugung von biologischem Gewebe (22, 24), insbesondere für die medizinische Behandlung und/oder für pharmakologische Untersuchungen, bei dem eine Unterlage (10) für Zellgewebe (22, 24) bereitgestellt wird, bei dem ein lebende Zellen enthaltenes Druckmedium bereitgestellt wird, bei dem das Druckmedium durch ein Drucksieb und/oder eine Druckschablone (14) auf die Unterlage (10) gedruckt wird, bei dem zwischen aufeinanderfolgenden Druckschritten ein Trocknungs- und Temperierungsschritt des aufgedruckten Druckmediums erfolgt und bei dem sich die gedruckten Zellen durch den Druck und/oder nach dem Druck zu Gewebe (22, 24) entwickeln.

2. Verfahren nach Anspruch 1, **dadurch gekennzeichnet, dass** das Druckmedium durch das Drucksieb und/oder die Druckschablone (14) auf die Unterlage (10) mit oder ohne Scaffold, Matrize und/oder strukturelles Gerüst gedruckt wird und/oder dass auf der Unterlage (10) strukturelle Gerüste, Matrizen und/oder Scaffolds vorgesehen sind und/oder dass strukturelle Gerüste, Matrizen und/oder Scaffolds gemeinsam mit dem Druckmedium gedruckt werden und/oder in diesem enthalten sind.

3. Verfahren nach Anspruch 1 oder 2, **dadurch gekennzeichnet, dass** das Druckmedium lagenweise gedruckt und/oder schichtweise aufgebaut wird und/oder dass eine einzelne Drucklage und/oder Druckschicht in einem einzigen Druckvorgang erzeugt wird und/oder dass eine Drucklage eine geringere Dicke in Druckaufbaurichtung aufweist als die Erstreckung der Drucklage quer zur Druckaufbaurichtung.

4. Verfahren nach einem der vorstehenden Ansprüche, **dadurch gekennzeichnet, dass** das Druckmedium durch mindestens einen Rakelvorgang und/oder mindestens eine Druckrakelbewegung, insbesondere durch eine Mehrzahl von Rakelvorgängen und/oder eine Mehrzahl von Druckrakelbewegungen, durch das Drucksieb und/oder die Druckschablone (14) hindurch und auf die Unterlage (10) befördert wird.

5. Verfahren nach einem der vorstehenden Ansprüche, **dadurch gekennzeichnet, dass** das Druckmedium durch mindestens einen Siebdruckvorgang, insbesondere durch eine Mehrzahl von Siebdruckvorgängen, auf die Unterlage (10) gedruckt wird und/oder dass das Drucken des Druckmediums auf die Unterlage (10) im Siebdruckverfahren erfolgt, insbesondere im 2D Siebdruckverfahren oder 3D-Siebdruckverfahren.

6. Verfahren nach einem der vorstehenden Ansprüche, **dadurch gekennzeichnet, dass** durch das Bedrucken der Unterlage (10) mit dem Druckmedium eine dreidimensionale Zellkultur und/oder eine dreidimensionale Zellagglomeration erzeugt wird und/oder dass sich nach dem Bedrucken der Unterlage (10) die jeweilige Zellkultur und/oder Zellagglomeration zu einem dreidimensionalen Gewebe (22, 24) und/oder einer dreidimensionalen Gewebestruktur entwickelt.

7. Verfahren nach einem der vorstehenden Ansprüche, **dadurch gekennzeichnet, dass** sich das gedruckte Druckmedium und/oder die gedruckten Zellen und/oder Zellagglomerationen durch den Druck oder nach dem Bedrucken zu Organgewebe, insbesondere Lebergewebe, Nierengewebe, Herzgewebe, Hautgewebe und/oder Muskelgewebe, und/oder zu einem teilweisen oder vollständigen Organ, insbesondere Leber, Niere, Herz und/oder Haut, bevorzugt zur Transplantation, und/oder dass sich das gedruckte Druckmedium und/oder die gedruckten Zellen und/oder Zellagglomerationen durch den Druck oder nach dem Bedrucken zu verzehrbarem Fleisch und/oder fleischhaltigem Lebensmittel entwickeln.

8. Verfahren nach einem der vorstehenden Ansprüche, **dadurch gekennzeichnet, dass** das Drucksieb und/oder die Druckschablone (14) zumindest einen Ausschnitt (18, 19) und/oder eine Mehrzahl von Ausschnitten (18, 19) für den Durchlass des Druckmediums aufweist, insbesondere getrennt voneinander ausgebildete Ausschnitte (18, 19), und/oder dass das Drucksieb und/oder die Druckschablone (14) wenigstens einen Ausschnitt (18, 19) mit ineinander übergehenden Abschnitten aufweist.

9. Verfahren nach einem der vorstehenden Ansprüche, **dadurch gekennzeichnet, dass** zum Drucken eines Gewebes (22, 24) unterschiedliche Drucksiebe und/oder Druckschablonen (14) eingesetzt werden, insbesondere um unterschiedlich geformte Drucklagen zu erzeugen, und/oder dass durch die unterschiedliche Formgebung verschiedener Drucksiebe und/oder Druckschablonen (14) komplexe dreidimensionale Gewebe (22, 24) und/oder Gewebestrukturen erzeugt werden, insbesondere Blutgefäße, Gefäßwände, Kanäle, strukturelle Gerüste, Matrizen und/oder Scaffolds.

10. Verfahren nach einem der vorstehenden Ansprüche, **dadurch gekennzeichnet, dass** zum Drucken unterschiedlicher Lagen unterschiedliche Druckmedien und/oder Druckmedien mit unterschiedlichen Zellen verwendet werden, insbesondere zur Erzeugung von Gewebe (22, 24) mit unterschiedlichen Lageneigenschaften und/oder zur Erzeugung von unterschiedlichen Hautlagen, und/oder dass in einer Druckaufbaurichtung eine Variation des Druckmediums vorgenommen wird.

11. Verfahren nach einem der vorstehenden Ansprüche, **dadurch gekennzeichnet, dass** innerhalb einer Drucklage, insbesondere entlang einer x/y-Richtung und/oder quer zu einer Druckaufbaurichtung, eine Gewebevariation erzeugt wird und/oder dass Gewebevariationen durch den Einsatz verschiedener Drucksiebe und/oder Druckschablonen (14) erzeugt werden, insbesondere verschiedene Drucksiebe und/oder Druckschablonen (14) pro Zelltyp und/oder Druckbereich.

12. Verfahren nach einem der vorstehenden Ansprüche, **dadurch gekennzeichnet, dass** das Druckmedium als Druckpaste und/oder niedrigviskose oder mittelviskose Nährflüssigkeit und/oder flüssige Suspension und/oder Sol-Gel Matrix ausgebildet ist und/oder dass das Druckmedium eine variable Viskosität aufweist und/oder dass die Viskosität des Druckmediums durch einen Trocknungsschritt und/oder Temperierungsschritt veränderbar ist und/oder dass das Druckmedium strukturviskos ist.

13. Verfahren nach einem der vorstehenden Ansprüche, **dadurch gekennzeichnet, dass** nach dem Drucken des Druckmediums ein Sol-Gel-Übergang erzeugt wird, insbesondere durch einen Trocknungs- und/oder Temperierungsschritt, und/oder dass nach einem erzeugten Sol-Gel-Übergang ein weiterer Druckvorgang vorgenommen wird.

14. Verfahren zur Erzeugung von biologischem Gewebe (22, 24), nach einem der vorstehenden Ansprüche, bei dem eine Unterlage (10) für Zellgewebe (22, 24) bereitgestellt wird, bei dem ein lebende Zellen enthaltenes Druckmedium mittels Siebdrucks auf die Unterlage (10) gedruckt wird und bei dem sich die gedruckten Zellen durch den Druck und/oder nach dem Druck zu Gewebe (22, 24) entwickeln.

## Claims

1. A method for producing biological tissue (22, 24), in particular for medical treatment and/or for pharmacological examinations, in which a support (10) for cell tissue (22, 24) is provided, in which a printing medium containing living cells is provided, in which the printing medium is printed through a printing screen and/or a printing stencil (14) onto the support (10), in which between successive printing steps a drying and tempering step of the imprinted printing medium is conducted and in which the printed cells develop into tissue (22, 24) through the printing and/or after the printing.

2. The method according to claim 1, **characterized in that** the printing medium is printed through the printing screen and/or the printing stencil (14) to the support (10) with or without scaffold, masters and/or structural framework and/or that structural frameworks, masters and/or scaffolds are provided on the support (10) and/or that structural frameworks, masters and/or scaffolds are jointly printed with the printing medium and/or contained therein.

3. The method according to claim 1 or 2, **characterized in that** the printing medium is printed in layers and/or constructed in layers and/or that a single printed layer is produced in a single printing operation and/or that a printed layer has a lower thickness in print build-up direction than the extent of the printed layer perpendicular to the print build-up direction.

4. The method according to any of the preceding claims, **characterized in that** the printing medium is conveyed by at least one doctor blade operation and/or at least one printing doctor blade movement, in particular through a plurality of doctor blade operations and/or a plurality of printing doctor blade movements, through the printing screen and/or the printing stencil (14) and onto the support (10).

5. The method according to any of the preceding claims, **characterized in that** the printing medium is printed through at least one screen-printing operation, in particular through a plurality of screen-printing operations, onto the support (10) and/or that the printing of the printing medium onto the support (10) is conducted in the screen-printing process, in particular in the 2D screen-printing process or 3D screen-printing process.

6. The method according to any of the preceding claims, **characterized in that** through the imprinting of the support (10) with the printing medium a three-dimensional cell culture and/or a three-dimensional cell agglomeration is produced and/or that after the imprinting of the support (10) the respective cell culture and/or cell agglomeration develops into a three-dimensional tissue (22, 24) and/or a three-dimensional tissue structure.

7. The method according to any of the preceding claims, **characterized in that,** through the printing or after the printing, the printed printing medium and/or the printed cells and/or cell agglomerations develop into organ tissue, in particular liver tissue, kidney tissue, heart tissue, skin tissue and/or muscle tissue, and/or into a partial or complete organ, in particular a liver, kidney, heart and/or skin, preferably for transplantation, and/or that, through the printing or after the printing the printed printing medium and/or the printed cells and/or cell agglomerations develop into consumable meat products and/or meat-containing food products.

8. The method according to any of the preceding claims, **characterized in that** the printing screen and/or the printing stencil (14) comprises at least one section (18, 19) and/or a plurality of sections (18, 19) for the passage of the printing medium, in particular sections (18, 19) formed separately from one another, and/or that the printing screen and/or the printing stencil (14) comprises at least one section (18, 19) with segments merging into one another.

9. The method according to any of the preceding claims, **characterized in that** different printing screens and/or printing stencils (14) are employed for printing a tissue (22, 24), in particular in order to produce differently formed printed layers, and/or that by means of the different designs of different printing screens and/or printing stencils (14) complex three-dimensional tissue (22, 24) and/or tissue structures are produced, in particular blood vessels, vessel walls, canals, structural frameworks, masters and/or scaffolds.

10. The method according to any of the preceding claims, **characterized in that** different printing media and/or printing media with different cells are used to print different layers, in particular for producing tissue (22, 24) with different layer properties and/or for producing different skin layers, and/or that a variation of the printing medium is performed in a print build-up direction.

11. The method according to any of the preceding claims, **characterized in that** within a printed layer, in particular along an x/y direction and/or perpendicular to a print build-up direction, a tissue variation is generated and/or **in that** tissue variations are generated by using different printing screens and/or printing stencils (14), in particular different printing screens and/or printing stencils (14) per cell type and/or printing region.

12. The method according to any of the preceding claims, **characterized in that** the printing medium is formed as printing paste and/or low viscosity or medium viscosity nutrient fluids and/or liquid suspension and/or Sol-Gel matrix and/or **in that** the printing medium has a variable viscosity and/or that the viscosity of the printing medium can be changed by a drying step and/or a tempering step and/or that the printing medium is structurally viscous.

13. The method according to any of the preceding claims, **characterized in that** after the printing of the printing medium a Sol-Gel transition is produced, in particular by a drying and or tempering step, and/or that after a produced Sol-Gel transition a further printing operation is performed.

14. A method for producing biological tissue (22, 24) according to any of the preceding claims, in which a support (10) for cell tissue (22, 24) is provided, in which a printing medium containing living cells is printed by means of screen printing onto the support (10) and in which the printed cells develop into tissue (22, 24) through the printing or after the printing.

## Revendications

1. Procédé de production d'un tissu biologique (22, 24), en particulier pour le traitement médical et/ou pour des examens pharmacologiques, dans lequel un support (10) est fourni pour des tissus cellulaires (22, 24), dans lequel un milieu d'impression qui contient des cellules vivantes est fourni, dans lequel le milieu d'impression est imprimé sur le support (10) par un tamis d'impression et/ou un pochoir d'impression (14), dans lequel une étape de séchage et de mise en température du milieu d'impression se produit entre des étapes d'impression qui se succèdent et dans lequel les cellules imprimées se développent en tissu (22, 24) par l'impression et/ou après l'impression.

2. Procédé selon la revendication 1, **caractérisé en ce que** le milieu d'impression est imprimé sur le support (10) par le tamis d'impression et/ou le pochoir d'impression (14) avec ou sans scaffold, matrice et/ou cadre structurel et/ou **en ce que** des cadres structurels, des matrices et/ou des scaffolds sont prévus sur le support (10) et/ou **en ce que** des cadres structurels, des matrices et/ou des scaffolds sont imprimés ensemble avec le milieu d'impression et/ou contenus dans celui-ci.

3. Procédé selon la revendication 1 ou 2, **caractérisé en ce que** le milieu d'impression est imprimé par couches et/ou monté par strates et/ou **en ce qu'**une unique couche d'impression et/ou strate d'impression est produite dans un unique processus d'impression et/ou **en ce qu'**une couche d'impression présente une épaisseur moindre dans la direction de développement d'impression que l'extension de la couche d'impression transversalement à la direction de développement d'impression.

4. Procédé selon l'une des revendications précédentes, **caractérisé en ce que** le milieu d'impression est acheminé par au moins un processus de racle et/ou au moins un mouvement de racle d'impression, en particulier par une pluralité de processus de racle et/ou une pluralité de mouvements de racle d'impression, au travers du tamis d'impression et/ou du pochoir d'impression (14) et jusque sur le support (10).

5. Procédé selon l'une des revendications précédentes, **caractérisé en ce que** le milieu d'impression est imprimé sur le support (10) par au moins un processus de tamis d'impression, en particulier par une pluralité de processus de tamis d'impression, et/ou **en ce que** l'impression du milieu d'impression se produit sur le support (10) dans le processus de tamis d'impression, en particulier dans le processus de tamis d'impression 2D ou le processus de tamis d'impression 3D.

6. Procédé selon l'une des revendications précédentes, **caractérisé en ce qu'une** culture de cellules tridimensionnelle et/ou une agglomération de cellules tridimensionnelle est produite par l'impression du support (10) avec le milieu d'impression, et/ou **en ce que** la culture de cellules et/ou agglomération de cellules respective se développe pour constituer un tissu (22, 24) tridimensionnel et/ou une structure tissulaire tridimensionnelle après l'impression du support (10).

7. Procédé selon l'une des revendications précédentes, **caractérisé en ce que** le milieu d'impression et/ou les cellules imprimées et/ou agglomérations de cellules se développent, par l'impression ou après l'impression, pour constituer des tissus organiques, en particulier des tissus hépatiques, des tissus rénaux, des tissus cardiaques, des tissus cutanés et/ou des tissus musculaires, et/ou pour constituer un organe partiel ou complet, en particulier un foie, un rein, un cœur et/ou une peau, de préférence pour une transplantation, et/ou **en ce que** le milieu d'impression imprimé et/ou les cellules imprimées et/ou agglomérations de cellules se développent, par l'impression ou après l'impression, pour constituer une viande comestible et/ou un aliment qui contient de la viande.

8. Procédé selon l'une des revendications précédentes, **caractérisé en ce que** le tamis d'impression et/ou le pochoir d'impression (14) présente au moins une section (18, 19) et/ou une pluralité de sections (18, 19) pour le passage du milieu d'impression, en particulier des sections (18, 19) conçues séparément les unes des autres, et/ou **en ce que** le tamis d'impression et/ou le pochoir d'impression (14) présente au moins une section (18, 19) avec des parties qui s'entremêlent.

9. Procédé selon l'une des revendications précédentes, **caractérisé en ce que** pour imprimer un tissu (22, 24) sont utilisés différents tamis d'impression et/ou pochoirs d'impression (14), en particulier pour produire des couches d'impression façonnées différemment, et/ou **en ce que**, par le façonnement différencié de différents tamis d'impression et/ou pochoirs d'impression (14), des tissus (22, 24) tridimensionnels et/ou structures tissulaires complexes sont produits, en particulier des vaisseaux sanguins, des parois vasculaires, des canaux, des cadres structurels, des matrices et/ou des scaffolds.

10. Procédé selon l'une des revendications précédentes, **caractérisé en ce que** des milieux d'impression différents et/ou des milieux d'impression avec des cellules différentes sont utilisés pour imprimer des couches différentes, en particulier pour produire des tissus (22, 24) avec des propriétés de couche différentes et/ou pour produire des couches cutanées différentes, et/ou **en ce qu'**une variation du milieu d'impression est réalisée dans une direction de développement d'impression.

11. Procédé selon l'une des revendications précédentes, **caractérisé en ce qu'une** variation tissulaire est produite à l'intérieur d'une couche d'impression, en particulier dans une direction x/y et/ou transversalement à une direction de développement d'impression, et/ou **en ce que** des variations tissulaires sont produites par l'insertion de différents tamis d'impression et/ou pochoirs d'impression (14), en particulier différents tamis d'impression et/ou pochoirs d'impression (14) par type de cellule et/ou zone d'impression.

12. Procédé selon l'une des revendications précédentes, **caractérisé en ce que** le milieu d'impression est conçu en tant que pâte d'impression et/ou liquide nutritif à basse viscosité ou à moyenne viscosité et/ou suspension liquide et/ou matrice sol-gel, et/ou **en ce que** le milieu d'impression présente une viscosité variable et/ou **en ce que** la viscosité du milieu d'impression peut être modifiée par une étape de séchage et/ou une étape de mise en température et/ou **en ce que** le milieu d'impression est d'une viscosité intrinsèque.

13. Procédé selon l'une des revendications précédentes, **caractérisé en ce qu'une** transition sol-gel est produite après l'impression du milieu d'impression, en particulier par une étape de séchage et/ou de mise en température, et/ou **en ce qu'**un autre processus d'impression est réalisé après la production d'une transition sol-gel.

14. Procédé de production d'un tissu biologique (22, 24) selon l'une des revendications précédentes, dans lequel un support (10) est fourni pour des tissus cellulaires (22, 24), dans lequel un milieu d'impression qui contient des cellules vivantes est imprimé sur le support (10) au moyen de tamis d'impression et dans lequel les cellules imprimées se développent, par l'impression et/ou après l'impression, pour constituer des cellules (22, 24).
